# EUROPEAN PATENT APPLICATION

(11) **EP 2 005 940 A2**
(43) Date of publication of application: **24.12.2008**
(21) Application number: 08252019.8
(22) Date of filing: 11.06.2008
(51) Int. Cl.: A61K 8/11, A61K 8/81, A61Q 13/00

(54) **Higher performance capsule particles**

(30) Priority: 12.06.2007 US 761458
(71) Applicant: International Flavors & Fragrances, Inc., New York, NY 10019 (US)
(72) Inventor: Lei, Yabin, Holmdel, New Jersey 07733 (US); Popplewell, Lewis Michael, Morganville, New Jersey 07751 (US)
(74) Representative: Lawrence, John

(57) **Abstract**

Capsule particles and a method of creating larger and robust capsule particles are disclosed. They have excellent storage stability and enhanced perfumer performance in a range of consumer products.

To be accompanied, when published, by Figure 1 of the drawings.

## Description

### Field of the Invention

This invention describes novel methods to produce high performance capsules particles that can contain active materials and polymeric adjuvants. The capsule particles produced are well suited for applications in a wide range of consumer products.

### Background of Invention

Encapsulation of active material, such as fragrances, is well known in the art. Encapsulation provides advantages to the fragrance product including the protection of the fragrance in the capsule core by a shell until the fragrance is intended to be delivered. In particular, capsules are often designed to deliver their contents at a desired time by the capsule shell being compromised at the desired time.

Various encapsulation techniques have been explored for the delivery of fragrance molecules. In many end applications, the encapsulation of aroma chemicals is a necessary, sometimes, mandatory process to protect the active ingredient from the harsh application environments they are in. It can also prevent the loss and premature release of active ingredients in the targeted application. The encapsulation of aroma chemicals can enable one to control and modulate the release profiles of aroma chemicals to achieve the desired perfumery effects and consumer benefits.

One of the critical issues for the application of a capsule particle in a consumer product base is the storage stability of capsule particle. For optimal sensory performance, a capsule particle should maintain its physical integrity and be able to retain the occluded materials for extended period of time when it is incorporated into a customer base.

Theoretically, a larger capsule should have increased diffusion length and be able to retain the encapsulated materials for longer period of time, leading to better storage stability. However, this increased stability predicted by theory cannot be assured in practical applications because the larger capsule will intrinsically have more structural defects. This can be especially true in concentrated surfactant solution that is known to be a very harsh medium for capsule particles because surfactant is a thermodynamic sinker for fragrance oils. Therefore, the creation of large capsule particles with good structural integrity, demonstrated storage stability and excellent perfumery benefit remains an active research area. The ability to produce such high quality capsule particle can significantly expand the application potential of aroma chemicals and bring to consumer an array of new products.

Fragrance capsules with physical dimensions in the microns to hundreds of micron regime have been mentioned in published patent literature, however, the capsule particles disclosed in the prior art with larger diameters are not able to maintain the integrity of a core/shell shape and actually collapse underneath their own weight because of intrinsic structural defects.

The use of water insoluble polymers in fragrance encapsulation was also described. But these polymers are normally solid materials and are utilized in a matrix type of encapsulation where the polymeric materials function solely as absorbents for fragrance oils. In all cases, the fragrance loaded core remains as a solid before being encapsulated.

The incorporation of polymeric material in the capsule core in the core/shell type of encapsulation has not been discussed. The adjuvant effects of these core polymers in forming stable large capsules have not been revealed. The potential consumer and perfumery benefits of larger capsule containing the liquid core polymers have never been published in the public domain.

It is thus the objectives of this invention to create larger and robust fragrance capsules with enhanced consumer and perfumery benefits.

### Summary of the Invention

It is an object of our invention to provide a larger and robust capsule particle that will have improved storage stability and enhanced perfumer performance in a range of consumer products.

In one embodiment, of the invention a capsule particle is prepared by the process of providing an oil phase containing fragrance oil and polymer adjuvant and an aqueous phase polymer solution then emulsifying the oil phase and the aqueous polymer solution and curing the emulsion at elevated temperature.

In another embodiment, the present invention provide capsule particle with about 1 to about 60% of oil phase, about 1 to about 20 % aqueous polymer solution with the remaining being water and a diameter from about 5 to about 2000 micron, more preferably 10 to 500 microns and most preferably 20 to 100 microns.

In yet another embodiment, the present invention provides a capsule particle with about 10 to about 45 % of oil phase, about 2 to about 10 % aqueous polymer solution with the remaining being water and a capsule particle with a diameter from about 5 to about 2000 micron, more preferably 10 to 500 microns and most preferably 20 to 100 microns.

In still another embodiment the capsule particle comprises a fragrance core composition with about 1 to 90% of the polymeric adjuvant, more preferably 5 to 50% of the polymeric adjuvant, and most preferably 10 to 30% of the polymeric adjuvant. The polymeric adjuvant either completely dissolves in the oil or is completely dispersed in the oil.

In yet another embodiment, the present invention provides higher performance capsule particles that can be used in a range of consumer products including, but not limited to, rinse conditioner, powder and liquid detergents, industrial and institutional cleaners, personal care, shampoo, conditioner, and body wash.

### Brief Description of the Drawings

Figure I is the TGA (thermogram metric analysis) thermogram of the same sample and was contrasted with that of a smaller capsule.

### Detailed Description of the Invention

The capsule particles disclosed by the present invention offer many advantages over existing capsule particle systems disclosed in the literature. The claimed capsule particles have robust physical stability and retain the encapsulated active material for extended periods of time in harsh application environments. The claimed capsule particle generates superb sensory and perfumery profiles appealing to the consumer. The capsule particle diameter of the present invention may be from about 5 to about 2000 micron, more preferably 10 to 500 microns and most preferably 20 to 100 microns.

The molecular weight of the polymeric adjuvant can vary from about 200 to about 200,000 Dalton, preferably from about 500 to about 50,000 Daltons, more preferably from about 1,000 to about 25,000, and most preferably from about 500 to about 15,000 Daltons. Liquid ingredients are preferred over solid ingredients.

The amount of polymeric adjuvant in the core can vary from about 1 to about 90%, preferably from about 5 to about 50% and most preferably from about 10% to about 30%.

The following list of polymeric adjuvants includes, but not limited, to the following groups:
1. polybutadiene homopolymers, copolymer and derivatives, functionalized polybutadiene polymers such as hydroxyl terminated polybutadiene resins manufactured by the Sartomer Company, Exton, PA. The functional groups can include hydroxyl, carboxylate, isocyanante, anhydride, epoxide and other chemical functionalities. The polymer backbone can be linear or branched. The functional groups can be in the primary, or secondary or tertiary position. Commericially available examples from Sartomer Company are: Poly bd^{®} R45HTLO, Poly bd^{®} R20LM, Krasol^{®} LBH 10000, Poly bd^{®} LF1, Poly bd^{®} LF2, Poly bd^{®} LF3, Poly bd^{®} 600E, Poly bd^{®} 605E, Krasol^{®} LBH 2000, Krasol^{®} LBH 3000, Krasol^{®} LBH 5000, Krasol^{®} LBH P-2000, Krasol^{®} LBH P-3000, Krasol^{®} LBH P-5000, Ricon^{®} 130, Ricon^{®} 131, Ricon^{®} 134, Ricon^{®} 142, Ricon^{®} P30D which is a polymer mixture dispersed on calcium silicate, Krasol^{®} LBD-2000, Krasol^{®} LBD-3000, Krasol^{®} NN-22, Krasol^{®} NN-23, Krasol^{®} NN-25, Poly bd^{®} 45CT, Poly bd^{®} 2000CT, and Poly bd^{®} 3000CT
2. Poly(maleic co-olefin), poly(maleic anhydride-alt-1-octadecene, Maleic anhydride/octadecene copolymer. The polymers can be dissolved in a suitable organic solvent such as, but not limited to, diethylhexyl sebacate, methyl acetyl ricinoleate, dimethylheptyl adipate and other suitable solvent. These polymeric materials are available as Stantive^{™} OMA-1 and OMA-2 from CasChem, Bayonne, NJ. They are also available as PA-18; PA 18 low color from Chevron Phillips, Woodland, TX.
3. Ethylene/propylene/styrene copolymer, butylene/ethylene/styrene copolymer and blends ethylene/propylene/styrene and butylene/ethylene/styrene copolymer at different ratios. The polymers and polymer blends may be pre-dispersed in a sutitable solvent such as mineral, hydrogenated polyisobutene, isopropyl palmitate, C12-15 alkyl benzoate, isohexadecane, isododecane, isononyl isononanoate. Commericially available examples from Penreco, Karns, PA are: Versagel^{®} M200, M500, M750, and M1600; Versagel^{®} ME500, ME750, ME1600, and ME2000; Versagel^{®} MP750 and MP1600; Versagel^{®} MC750 and MC1600; Versagel^{®} ML750 and ML1600; Versagel^{®} MD750 and MD1600; Versagel^{®} MN750 and MN1600. The polymers and polymer blends may also be pre-dispersed in any other suitable organic solvemts that can be identified by those skilled in the art.
4. Ester terminated polyamides (ETPA), ester terminated poly(ester-amide)(ETPEA), tertiary amide terminated polyamides (ATPA), polyalkyleneoxy terminated polyamides (PAOPA), and polyether polyamide (PEPA). The materials may include UNICLEAR^{®} 100VG, SYLVACLEAR^{®} C75V, SYLVACLEAR^{®} A200V, SYLVACLEAR^{®} A2614V, SYLVACLEAR^{®} AF1900V, SYLVACLEAR^{®} WF1500V, and SYLVACLEAR^{®} PA1200V. The materials may be obtained from Arizona Chemicals, Jacksonville, FL. Dilutions of such polymers with various non-polar organic solvents may also be used.
5. Hydroxyalkylcellulose such as hydroxyethyl cellulose, hydroxypropyl cellulose, hydroxylpropyl methylcellulose; cellulose ester such as cellulose acetate and cellulose acetate butyrate. These materials can be obtained from the Dow Chemical Company, Midland, MI, or Eastman Chemicals, Kingsport, TN, and Hercules, Wilmington, DE. Examples are ETHOCEL Std. 4, 7, 10, 14, 20, 45, 100 Premium, Premium FP, NF available from Dow; CA-320S (Cellulose Acetate), CAB-171-15( cellulose acetate butyrate) from Eastman; and Polysurf^{®} (cetyl hydroxyethylcellulose), Kucel hydroxylpropylcellulose (HPC), and AQUALON^{®} ethylcellulose (EC) from Hercules Chemical.
6. Dimeric, trimeric, and polymeric fatty acids and the materials are available from the Arizona Chemicals, Jacksonville, FL. The materials can be selected from Unidyme^{®} or Century^{®} product line. Examples are Unidyme^{®} and 10, 12, 14R, T-17, 18, T-18, 22, 40; Century^{®} D-75, D-77, D-78, 1156, 7001; and Sylvablend 7002.
7. Hydrophobic poly-propylene oxide-co-poly-ethylene oxide (EO-PO) polymer. The polymers can have either random or block structure. These polymers can also pre-disolved in a solvent and be used.
8. Blends of castor oil, hydrogenated and partially hydrogenated castor oil such as the Castolatum^{™} from Caschem, Bayonne, NJ.

An optional solid core material which can be used in combination with the polymer adjuvant can be selected from a non-limiting list of metal oxides, organic and inorganic clays that can be employed as core materials. Specific example may selected from, but not limited to, titanium dioxide (TiO₂), zinc oxide (ZnO₃), ferric oxide (Fe₂O₃), silica and other metal oxides. They should have specific density of greater than unity. In addition, hydrophobically modified metal oxides are preferred. Examples of hydrophobically modified metal oxides include Uvinul TiO₂, Z-COTE HP1, Z-COTE MAX, T-Lite SF, T-Lite SF-S, and T-Lite MAX manufactured by BASF; Aerosil R812, Aesosil R972/R94 from Degussa; and Ti-Pure^{®} R-700 and Ti-Select^{™} TS-6200 from Dupont.

The optional solid core material can be present in an amount so that the core of the capsule particle is still liquid. Thus, the system may contain solid core material from about 0 to about 20%, preferably from about 1 to about 10% and most preferably from 2 % about to about 6%.

The larger fragrance capsules provided in one embodiment of the invention can be prepared by various processes. In one embodiment the polymer adjuvant is pre-blended with the fragrance oil under slight shear to form homogenous solution. Then the encapsulating polymer is pre-formed with suitable monomers. The core material is then added into the aqueous solution of the coating polymer at the desired fragrance concentration under constant agitation using an overhead mixer. The formation of capsule is monitored with a microscope and the stirring speed can be altered to generate capsules of different physical dimension. The polymerization reaction is allowed to proceed further under constant agitation at elevated temperature and is stopped after the reaction or encapsulation is complete. The collected slurry can be used in any desired application.

In another embodiment, the oil phase composition of fragrance oil and polymer adjuvant can be directly blended into the aqueous polymer solution under shearing. The polymerization reaction is allowed to proceed further under constant agitation at elevated temperature. The polymerization reaction is stopped after the reaction is complete. The progress of reaction and encapsulation of the core materials can be monitored by optical microscope. The collected capsule particles can be used in any desired application.

The optional solid core ingredient such as titanium oxide and silica oxide can be pre-blended in the active material such as fragrance oil which is mixed with the polymeric adjuvant at a later stage. This multi-component core is encapsulated similarly as the fragrance oil.

Fragrance capsules prepared by the current application can be readily incorporated into consumer products such as rinse conditioner, liquid and powder detergent, cleaners, personal care, hair care, sun screen formulation, liquid makeup, and textile.

The benefits of fragrance capsule can be demonstrated by perfumery benefits they bring into consumer products. For examples, to evaluate their benefits in rinse conditioner (RC) or liquid detergent, the fragrance capsule can be blended into the RC solution. The solution can then be used to conduct a laundry experiment using commercially available towels. The efficacy of a consumer product containing capsule can be measured by the perfumery intensity before any physical force is applied to the fabric, the pre-rubbing intensity (Iₚᵣₑ); and when the fabric is subject to some sort of mechanical perturbation such as physical rubbing, the post-rubbing intensity (Iₚₒₛₜ); and the ratio of (Iₚₒₛₜ)/(Iₚᵣₑ). As it is common for consumers or fragrance users to expect good and strong "smell" when they are in contact with a perfumed object, it is very desirable for an encapsulated fragrance to generate as higher a pre-rubbing intensity as possible and to have an optimal overall intensity.

We have surprisingly discovered that the larger fragrance capsules prepared from the current invention have significantly increased the Iₚᵣₑ intensity in the consumer products containing these capsules while maintaining the high overall fragrance intensity. The finished product has a more favorable release profile and can delivery the perfumery benefits at all stages of application.

A physical method to evaluate the ability of fragrance capsule to hold onto the occluded material is thermo gravimetrical analysis (TGA) where the weight loss of material is measured as a function of temperature. It is expected that a stronger capsule that is able to hold its content more tightly will have a higher onset temperature at which the release of the encapsulated material begins to occur. Thus TGA offers a very valuable tool for assessing the relative strength or the ability of capsule systems to retain the encapsulated material.

We have surprisingly discovered that the larger fragrance capsule prepared according to an embodiment of the invention has a much higher onset temperature at which fragrance release begins to occur than a smaller capsule cured at the same temperature. This strongly suggests that the larger capsules prepared by our current invention are more robust system and are more resistant towards fragrance leaching, suggestive of the fact that the system has excellent thermal stability.

Since the creation of larger and robust fragrance capsule using polymer adjuvants has not been disclosed before, the release profile of capsule particles are not known. Furthermore, there are no reported studies or disclosures specifically examining the effect of capsule size on the sensory and perfumery performance of fragrance capsules. The current invention provides the means of creating larger fragrance capsules and offers the possibility to engineer the release profile of the fragrance ingredient from the capsule particle for optimum consumer appeal.

Encapsulation of active material such as fragrances is known in the art, see for example U.S. Patent Nos. 2,800,457, 3,870,542, 3,516,941, 3,415,758, 3,041,288, 5,112,688, 6,329,057, and 6,261,483. Another discussion of fragrance encapsulation is found in the Kirk-Othmer Encyclopedia.

The encapsulating polymers include those formed from, acrylates, acrylamide, acrylate-co-acrylamide, melamine-formaldehyde or urea-formaldehyde condensates, as well as similar types of aminoplasts. The encapsulating material or the wall forming materials can be selected from a wide range of polymers, co-polymers, cross-linked polymers including melamine formaldehyde resins, polyureathane polymers and resins. The polymer and copolymers can also be selected from polyacrylates, polyacrylamides, poly(acrylamide-co-acrylate), polyvinyl alcohol, poly(ethylene-co-vinyl acetate) (EVA), poly(vinylpyrrolidone-co-styrene), poly(ethylene oxide-co-propylene oxide), poly(styrene-co-maleic anhydride), poly(ethylene-alt-maleic anhydride) (EMA), and salt of poly(methacryloyloxyethyly).

Other wall forming materials include polyurethane, polysiloxanes, polyurea, polyamide, polyimide, polyvinyl alcohol, polyanhydride, polyolefin, polysulfone, polysaccaharide, protein, polylactide (PLA), polyglycolide (PGA), polyorthoester, polyphosphazene, silicone, lipid, modified cellulose, gums, polystyrene, and polyesters or combinations of these materials. Other polymeric materials that are functional are ethylene maleic anyhydride copolymer, styrene maleic anyhydride copolymer, ethylene vinyl acetate copolymer and lactide glycolide copolymer. Bio-polymers that are derived from alginate, chitosan, collegen, dextran, gelatin, and starch can also be used as the encapsulating materials. Additionally, microcapsules made via the simple or complex coacervation of gelatin are also preferred for use with the coating.

The ratio of wall forming polymer to that of core by weight independent of the slurry composition falls in the range of about 1:100 to about 50:1, more preferable in the range of about 1:50 to about 25:1, even more preferable in the range of about 1:25 to about 10:1 and most preferably of about 1:10 to about 5:1.

A representative process used for aminoplast encapsulation is disclosed in U.S. Patent No. 3,516,941 though it is recognized that many variations with regard to material and process steps are possible. A representative process used for gelatin encapsulation is disclosed in U.S. Patent No, 2,800,457 though it is recognized that many variations with regard to material and process steps are possible. Both of these processes are discussed in the context of fragrance encapsulation for use in consumer products in U.S. Patent Nos. 4,145,184 and 5,112,688 respectively.

Well known material such as solvents, surfactants, emulsifiers, and the like can be used in addition to the polymers described throughout the invention to encapsulate the active material such as fragrance without departing from the scope of the present invention. It is understood that the term encapsulated is meant to mean that the active material is substantially covered in its entirety. Encapsulation can provide pore vacancies or interstitial openings depending on the encapsulation techniques employed. More preferably the entire active material portion of the present invention is encapsulated.

Fragrance capsules known in the art consists of a core of various ratios of fragrance and solvent material, a wall or shell comprising a three-dimensional cross-linked network of an aminoplast resin, more specifically a substituted or un-substituted acrylic acid polymer or co-polymer cross-linked with a urea-formaldehyde pre-condensate or a melamine-formaldehyde pre-condensate.

Microcapsule formation using mechanisms similar to the foregoing mechanism, using (i) melamine-formaldehyde or urea-formaldehyde pre-condensates and (ii) polymers containing substituted vinyl monomeric units having proton-donating functional group moieties (e.g. sulfonic acid groups or carboxylic acid anhydride groups) bonded thereto is disclosed in U.S. Patent 4,406,816 (2-acrylamido-2-methyl-propane sulfonic acid groups), UK published Patent Application GB 2,062,570 A (styrene sulfonic acid groups) and UK published Patent Application GB 2,006,709 A (carboxylic acid anhydride groups).

The cross-linkable acrylic acid polymer or co-polymer microcapsule shell wall precursor has a plurality of carboxylic acid moieties, to wit:

The cross-linkable acrylic acid polymer or co-polymer microcapsule shell wall precursor has a plurality of carboxylic acid moieties, to wit: 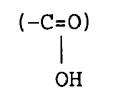 and is preferably one or a blend of the following:
(i) an acrylic acid polymer;
(ii) a methacrylic acid polymer;
(iii) an acrylic acid-methacrylic acid co-polymer;
(iv) an acrylamide-acrylic acid co-polymer;
(v) a methacrylamide-acrylic acid co-polymer;
(vi) an acrylamide-methacrylic acid co-polymer;
(vii) a methacrylamide-methacrylic acid co-polymer;
(viii) a C₁-C₄ alkyl acrylate-acrylic acid co-polymer;
(ix) a C₁-C₄ alkyl acrylate-methacrylic acid co-polymer;
(x) a C₁-C₄ alkyl methacrylate-acrylic acid co-polymer;
(xi) a C₁-C₄ alkyl methacrylate-methacrylic acid co-polymer;
(xii) a C₁-C₄ alkyl acrylate-acrylic acid-acrylamide co-polymer;
(xiii) a C₁-C₄ alkyl acrylate-methacrylic acid-acrylamide co-polymer;
(xiv) a C₁-C₄ alkyl methacrylate-acrylic acid-acrylamide co-polymer;
(xv) a C₁-C₄ alkyl methacrylate-methacrylic acid-acrylamide co-polymer;
(xvi) a C₁-C₄ alkyl acrylate-acrylic acid-methacrylamide co-polymer;
(xvii) a C₁-C₄ alkyl acrylate-methacrylic acid-methacrylamide co-polymer;
(xviii) a C₁-C₄ alkyl methacrylate-acrylic acid-methacrylamide co-polymer; and
(xix) a C₁-C₄ alkyl methacrylate-methacrylic acid-methacrylamide co-polymer;
and more preferably, an acrylic acid-acrylamide copolymer.

When substituted or un-substituted acrylic acid co-polymers are employed in the practice of our invention, in the case of using a co-polymer having two different monomeric units, e.g. acrylamide monomeric units and acrylic acid monomeric units, the mole ratio of the first monomeric unit to the second monomeric unit is in the range of from about 1:9 to about 9:1, preferably from about 3:7 to about 7:3. In the case of using a co-polymer having three different monomeric units, e.g. ethyl methacrylate, acrylic acid and acrylamide, the mole ratio of the first monomeric unit to the second monomeric unit to the third monomeric unit is in the range of 1:1:8 to about 8:8:1, preferably from about 3:3:7 to about 7:7:3.

The molecular weight range of the substituted or un-substituted acrylic acid polymers or co-polymers useful in the practice of our invention is from about 5,000 to about 1,000,000, preferably from about 10,000 to about 100,000. The substituted or un-substituted acrylic acid polymers or co-polymers useful in the practice of our invention may be branched, linear, star-shaped, dendritic-shaped or may be a block polymer or copolymer, or blends of any of the aforementioned polymers or copolymers.

Such substituted or un-substituted acrylic acid polymers or co-polymers may be prepared according to any processes known to those skilled in the art, for example, U.S. Patent 6,545,084.

The urea-formaldehyde and melamine-formaldehyde pre-condensate microcapsule shell wall precursors are prepared by means of reacting urea or melamine with formaldehyde where the mole ratio of melamine or urea to formaldehyde is in the range of from about 10:1 to about 1:6, preferably from about 1:2 to about 1:5 . For purposes of practicing our invention, the resulting material has a molecular weight in the range of from about 150 to about 3000. The resulting material may be used 'as-is' as a crosslinking agent for the aforementioned substituted or un-substituted acrylic acid polymer or copolymer or it may be further reacted with a C1-C6 alkanol, e.g. methanol, ethanol, 2-propanol, 3-propanol, 1-butanol , 1-pentanol or 1-hexanol, thereby forming a partial ether where the mole ratio of melamine or urea:formaldehyde:alkanol is in the range of 1:(0.1 - 6):(0.1-6). The resulting ether moiety-containing product may by used 'as-is' as a cross-linking agent for the aforementioned substituted or un-substituted acrylic acid polymer or copolymer, or it may be self-condensed to form dimers, trimers and/or tetramers which may also be used as cross-linking agents for the aforementioned substituted or un-substituted acrylic acid polymers or co-polymers. Methods for formation of such melamine-formaldehyde and urea-formaldehyde pre-condensates are set forth in U.S. Patent 3,516,846, U.S. Patent 6,261,483, and Lee et al. J. Microencapsulation, 2002, Vol. 19, No. 5, pp 559-569, "Microencapsulation of fragrant oil via in situ polymerization: effects of pH and melamine-formaldehyde molar ratio". Examples of urea-formaldehyde pre-condensates useful in the practice of our invention are URAC 180 and URAC 186, trademarks of Cytec Technology Corp. of Wilmington, Delaware 19801, U.S.A. Examples of melamine-formaldehyde pre-condensates useful in the practice of our invention are CYMEL U-60, CYMEL U-64 and CYMEL U-65, trademarks of Cytec Technology Corp. of Wilmington, Delaware 19801, U.S.A. In the practice of our invention it is preferable to use as the precondensate for cross-linking the substituted or un-substituted acrylic acid polymer or co-polymer. The melamine-formaldehyde pre-condensate having the structure: 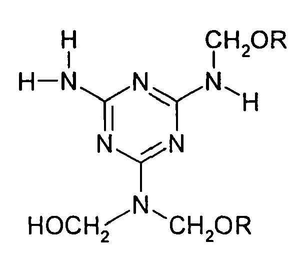 wherein each of the R groups are the same or different and each represents hydrogen or C1-C6 lower alkyl, e.g. methyl, ethyl, 1-propyl, 2-propyl, 1-butyl, 2-butyl, 2-methyl-1-propyl, 1-pentyl, 1-hexyl and/or 3-methyl-1-pentyl.

The range of mole ratios of urea-formaldehyde precondensate or melamine-formaldehyde pre-condensate: substituted or un-substituted acrylic acid polymer or co-polymer is in the range of from about 9:1 to about 1:9, preferably from about 5:1 to about 1:5 and most preferably from about 2:1 to about 1:2.

In another embodiment of the invention, microcapsules with polymer(s) comprising primary and/or secondary amine reactive groups or mixtures thereof and crosslinkers as disclosed in commonly assigned U.S. Patent Application No. 11/123,898.

The amine polymers can possess primary and/or secondary amine functionalities and can be of either natural or synthetic origin. Amine containing polymers of natural origin are typically proteins such as gelatin and albumen, as well as some polysaccharides. Synthetic amine polymers include various degrees of hydrolyzed polyvinyl formamides, polyvinylamines, polyallyl amines and other synthetic polymers with primary and secondary amine pendants. Examples of suitable amine polymers are the Lupamin series of polyvinyl formamides (available from BASF). The molecular weights of these materials can range from 10,000 to 1,000,000.

The polymers containing primary and/or secondary amines can be used with any of the following comonomers in any combination:
1. Vinyl and acrylic monomers with:
   a. alkyl, aryl and silyl substituents;
   b. OH, COOH, SH, aldehyde, trimonium, sulfonate, NH2, NHR substiuents;
   c. vinyl pyridine, vinyl pyridine-N-oxide, vinyl pyrrolidon
2. Cationic monomers such as dialkyl dimethylammonium chloride, vinyl imidazolinium halides, methylated vinyl pyridine, cationic acrylamides and guanidine-based monomers
3. N-vinyl formamide and any mixtures thereof. The ratio amine monomer/total monomer ranges from 0.01 - 0.99, more preferred from 0.1 - 0.9.

The following represents a general formula for the amine-containing polymer material: 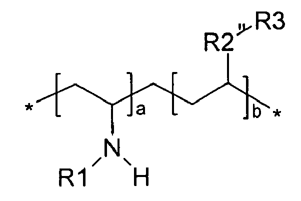 wherein R is a saturated or unsaturated alkane, dialkylsiloxy, dialkyloxy, aryl, alkylated aryl, and that may further contain a cyano, OH, COOH, NH2, NHR, sulfonate, sulphate, -NH2, quaternized amines, thiols, aldehyde, alkoxy, pyrrolidone, pyridine, imidazol, imidazolinium halide, guanidine, phosphate, monosaccharide, oligo or polysaccharide.
R1 is H, CH3, (C=O)H, alkylene, alkylene with unsaturated C-C bonds, CH2-CROH, (C=O)-NH-R, (C=O)-(CH2)n-OH, (C=O)-R, (CH2)n-E, -(CH2-CH(C=O))n-XR, -(CH2)n-COOH, -(CH2)n-NH2, -CH2)n-(C=O)NH2, E is an electrophilic group; wherein a and b are integers or average numbers (real numbers) from about 100-25,000.
R2 can be nonexistent or the functional group selected from the group consisting of -COO-, -(C=O)-, -O-, -S-, -NH-(C=O)-,-NR1-, dialkylsiloxy, dialkyloxy, phenylene, naphthalene, alkyleneoxy. R3 can be the same or selected from the same group as R1.

Additional copolymers with amine monomers are provided having the structure:

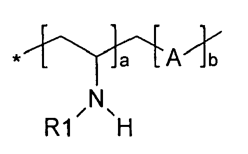

R1 is H, CH3, (C=O)H, alkylene, alkylene with unsaturated C-C bonds, CH2-CROH, (C=O)-NH-R, (C=O)-(CH2)n-OH, (C=O)-R, (CH2)n-E, -(CH2-CH(C=O))n-XR, -(CH2)n-COOH, -(CH2)n-NH2, -CH2)n-(C=O)NH2, E is an electrophilic group; wherein a and b are integers or average numbers (real numbers) from about 100-25,000; wherein R is a saturated or unsaturated alkane, dialkylsiloxy, dialkyloxy, aryl, alkylated aryl, and that may further contain a cyano, OH, COOH, NH2, NHR, sulfonate, sulphate, -NH2, quaternized amines, thiols, aldehyde, alkoxy, pyrrolidone, pyridine, imidazol, imidazolinium halide, guanidine, phosphate, monosaccharide, oligo or polysaccharide.

The comonomer, represented by A, can contain an amine monomer and a cyclic monomer wherein A can be selected from the group consisting of aminals, hydrolyzed or non-hydrolyzed maleic anhydride, vinyl pyrrolidine, vinyl pyridine, vinyl pyridine-N-oxide, methylated vinyl pyridine, vinyl naphthalene, vinyl naphthalene-sulfonate and mixtures thereof.

When A is an aminal the following general structure can represent the aminal: 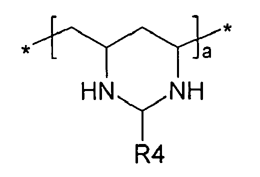 wherein R4 is selected from the group consisting of H, CH3, (C=O)H, alkylene, alkylene with unsaturated C-C bonds, CH2-CROH, (C=O)-NH-R, (C=O)-(CH2)n-OH, (C=O)-R, (CH2)n-E, -(CH2-CH(C=O))n-XR, -(CH2)n-COOH, -(CH2)n-NH2, -CH2)n-(C=O)NH2, E is an electrophilic group; wherein R is a saturated or unsaturated alkane, dialkylsiloxy, dialkyloxy, aryl, alkylated aryl, and that may further contain a cyano, OH, COOH, NH2, NHR, sulfonate, sulphate, -NH2, quaternized amines, thiols, aldehyde, alkoxy, pyrrolidone, pyridine, imidazol, imidazolinium halide, guanidine, phosphate, monosaccharide, oligo or polysaccharide.

In addition instead of amine-containing polymers it is possible to utilize amine-generating polymers that can generate primary and secondary amines during the microcapsule formation process as disclosed in commonly assigned U.S. Patent Application No. 11/123,898.

The crosslinkers can be selected from the group consisting of aminoplasts, aldehydes such as formaldehyde and acetaldehyde, dialdehydes such as glutaraldehyde, epoxy, active oxygen such as ozone and OH radicals, poly-substituted carboxylic acids and derivatives such as acid chlorides, anyhydrides, isocyanates, diketones, halide-substituted, sulfonyl chloride-based organics, inorganic crosslinkers such as Ca2+, organics capable of forming azo, azoxy and hydrazo bonds, lactones and lactams, thionyl chloride, phosgene, tannin/tannic acid, polyphenols and mixtures thereof. Furthermore, processes such as free radical and radiation crosslinking can be used according to the present invention. Examples of free radical initiators are benzoyl peroxide, sodium persulfate, azoisobutylnitrile (AIBN) and mixtures thereof.

With respect to the crosslinker, wall properties are influenced by two factors: the degree of crosslinking and the hydrophobic or hydrophilic nature of the crosslinker. The quantity and reactivity of the crosslinker determine the degree of crosslinking. The degree of crosslinking influences the microcapsule wall permeability by forming physical barriers towards diffusion. Walls made from crosslinkers possessing low-reactive groups will have smaller degrees of crosslinking than walls made from high-reactive crosslinkers. If a high degree of crosslinking is desired from a low-reactive crosslinker, more is added. If a low degree of crosslinking is desired from a high-reactive crosslinker then less is added. The nature and quantity of the crosslinker can also influence the hydrophobicity/hydrophilicity of the wall. Some crosslinkers are more hydrophobic than others and these can be used to impart hydrophobic qualities to the wall, with the degree of hydrophobicity directly proportional to the quantity of crosslinker used.

The degree of crosslinking and degree of hydrophobicity can result from a single crosslinker or a combination of crosslinkers. A crosslinker that is highly reactive and hydrophobic can be used to create microcapsule walls with a high degree of crosslinking and a hydrophobic nature. Single crosslinkers that possess both these qualities are limited and thus crosslinker blends can be employed to exploit these combinations. Crosslinkers possessing high reactivities but low hydrophobicities can be used in combination with a low reactive, high hydrophobicity crosslinker to yield walls with high degrees of crosslinking and high hydrophobicity. Suitable crosslinkers are disclosed in commonly assigned U.S. Patent Application No. 11/123,898.
(A)Copolymers containing primary and/or secondary amine. When amine-containing polymers are employed in the practice of the invention, in the case of using a co-polymer having two different monomeric units, e.g. Lupamin 9030 (copolymer of vinyl amine and vinyl formamide), the mole ratio of the first monomeric unit to the second monomeric unit is in the range of from about 0.1:0.9 to about 0.9:0.1, preferably from about 1:9 to about 9:1. In the case of using a co-polymer having three different monomeric units, e.g. a copolymer of vinyl amine, vinyl formamide and acrylic acid, the mole ratio of the reactive monomer (i.e. vinyl amine + acrylic acid) in the total polymer ranging from about 0.1 to about 0.9, more preferably from about 1 to about 9.
(B) Branched amine containing polymers such as ethylene imines (Lupasol series of BASF) and ethoxylated ethylene imines.
(C) Mixtures of amine containing polymers and other polymers that contain other reactive groups such as COOH, OH, and SH.

The molecular weight range of the substituted or un-substituted amine-containing polymers or co-polymers and mixtures thereof, useful in the practice of our invention is from about 1,000 to about 1,000,000, preferably from about 10,000 to about 500,000. The substituted or un-substituted amine-containing polymers or co-polymers useful in the practice of our invention may be branched, linear, star-shaped, graft, ladder, comb/brush, dendritic-shaped or may be a block polymer or copolymer, or blends of any of the aforementioned polymers or copolymers. Alternatively, these polymers may also possess thermotropic and/or lyotropic liquid crystalline properties.

Particle and microcapsule diameter can vary from about 1 micron to about 2000 microns, preferably from about 5 microns to about 500 microns and most preferably from about 10 to about 200 microns. The microcapsule distribution can be narrow, broad, or multi-modal. Each modal of the multi-modal distributions may be composed of different types of microcapsule chemistries.

Once the fragrance material is encapsulated a cationically charged water-soluble polymer may be applied to the fragrance encapsulated polymer. This water-soluble polymer can also be an amphoteric polymer with a ratio of cationic and anionic functionalities resulting in a net total charge of zero and positive, i.e., cationic. Those skilled in the art would appreciate that the charge of these polymers can be adjusted by changing the pH, depending on the product in which this technology is to be used. Any suitable method for coating the cationically charged material onto the encapsulated fragrance material can be used. The nature of suitable cationically charged polymers for assisted microcapsule delivery to interfaces depends on the compatibility with the microcapsule wall chemistry since there has to be some association to the microcapsule wall. This association can be through physical interactions, such as hydrogen bonding, ionic interactions, hydrophobic interactions, electron transfer interactions or, alternatively, the polymer coating could be chemically (covalently) grafted to the microcapsule or particle surface. Chemical modification of the microcapsule or particle surface is another way to optimize anchoring of the polymer coating to microcapsule or particle surface. Furthermore, the microcapsule and the polymer need to want to go to the desired interface and, therefore, need to be compatible with the chemistry (polarity, for instance) of that interface. Therefore, depending on which microcapsule chemistry and interface (e.g., cotton, polyester, hair, skin, wool) is used the cationic polymer can be selected from one or more polymers with an overall zero (amphoteric: mixture of cationic and anionic functional groups) or net positive charge, based on the following polymer backbones: polysaccharides, polypeptides, polycarbonates, polyesters, polyolefinic (vinyl, acrylic, acrylamide, poly diene), polyester, polyether, polyurethane, polyoxazoline, polyamine, silicone, polyphosphazine, olyaromatic, poly heterocyclic, or polyionene, with molecular weight (MW) ranging from about 1,000 to about 1000,000,000, preferably from about 5,000 to about 10,000,000. As used herein molecular weight is provided as weight average molecular weight. Optionally, these cationic polymers can be used in combination with nonionic and anionic polymers and surfactants, possibly through coacervate formation.

A more detailed list of cationic polymers that can be used to is provided below:
Polysaccharides include but are not limited to guar, alginates, starch, xanthan, chitosan, cellulose, dextrans, arabic gum, carrageenan, hyaluronates. These polysaccharides can be employed with:
   (a) cationic modification and alkoxy-cationic modifications, such as cationic hydroxyethyl, cationic hydroxy propyl. For example, cationic reagents of choice are 3-chloro-2-hydroxypropyl trimethylammonium chloride or its epoxy version. Another example is graft-copolymers of polyDADMAC on cellulose like in Celquat L-200 (Polyquaternium-4), Polyquaternium-10 and Polyquaternium-24, commercially available from National Starch, Bridgewater, N.J.;
   (b) aldehyde, carboxyl, succinate, acetate, alkyl, amide, sulfonate, ethoxy, propoxy, butoxy, and combinations of these functionalities. Any combination of Amylose and Mylopectin and overall molecular weight of the polysaccharide; and
   (c) any hydrophobic modification (compared to the polarity of the polysaccharide backbone).

The above modifications described in (a), (b) and (c) can be in any ratio and the degree of functionalization up to complete substitution of all functionalizable groups, and as long as the theoretical net charge of the polymer is zero (mixture of cationic and anionic functional groups) or preferably positive. Furthermore, up to 5 different types of functional groups may be attached to the polysaccharides. Also, polymer graft chains may be differently modified than the backbone. The counterions can be any halide ion or organic counter ion. As disclosed in U.S. Letter for Patent Nos. 6,297,203 and U.S. 6,200,554.

Another source of cationic polymers contain protonatable amine groups so that the overall net charge is zero (amphoteric: mixture of cationic and anionic functional groups) or positive. The pH during use will determine the overall net charge of the polymer. Examples are silk protein, zein, gelatin, keratin, collagen and any polypeptide, such as polylysine.

Further cationic polymers include poly vinyl polymers, with up to 5 different types of monomers, having the monomer generic formula -C(R2) (R1)-CR2R3-. Any co-monomer from the types listed in this specification may also be used. The overall polymer will have a net theoretical positive charge or equal to zero (mixture of cationic and anionic functional groups). Where R1 is any alkanes from C1-C25 or H; the number of double bonds ranges from 0-5. Furthermore, R1 can be an alkoxylated fatty alcohol with any alkoxy carbon-length, number of alkoxy groups and C1-C25 alkyl chain length. R1 can also be a liquid crystalline moiety that can render the polymer thermotropic liquid crystalline properties, or the alkanes selected can result in side-chain melting. In the above formula R2 is H or CH3; and

R3 is -Cl, -NH2 (i.e., poly vinyl amine or its copolymers with N-vinyl formamide. These are sold under the name Lupamin 9095 by BASF Corporation), -NHR1, -NR1R2, -NR1R2 R6 (where R6 = R1, R2, or -CH2-COOH or its salt), -NH-C(O)-H, -C(O)-NH2 (amide), -C(O)-N(R2)(R2')(R2"), -OH, styrene sulfonate, pyridine, pyridine-N-oxide, quaternized pyridine, imidazolinium halide, imidazolium halide, imidazol, piperidine, pyrrolidone, alkyl-substituted pyrrolidone, caprolactam or pyridine, phenyl-R4 or naphthalene-R5 where R4 and R5 are R1, R2, R3, sulfonic acid or its alkali salt -COOH, -COO- alkali salt, ethoxy sulphate or any other organic counter ion. Any mixture or these R3 groups may be used. Further suitable cationic polymers containing hydroxy alkyl vinyl amine units, as disclosed in U.S. Patent No 6,057,404.

Another class of material is polyacrylates, with up to 5 different types of monomers, having the monomer generic formula: -CH(R1)-C(R2)(CO-R3-R4)-. Any co-monomer from the types listed in this specification may also be used. The overall polymer will have a net theoretical positive charge or equal to zero (mixture of cationic and anionic functional groups). In the above formula R1 is any alkane from C1-C25 or H with number of double bonds from 0-5,aromatic moieties, polysiloxane, or mixtures thereof. Furthermore, R1 can be an alkoxylated fatty alcohol with any alkoxy carbon-length, number of alkoxy groups and C1-C25 alkyl chain length. R1 can also be a liquid crystalline moiety that can render the polymer thermotropic liquid crystalline properties, or the alkanes selected can result in side-chain melting. R2 is H or CH3; R3 is alkyl alcohol C1-25 or an alkylene oxide with any number of double bonds, or R3 may be absent such that the C=O bond is (via the C-atom) directly connected to R4. R4 can be: -NH2, NHR1, -NR1R2, -NR1R2 R6 (where R6 = R1, R2, or -CH2-COOH or its salt), -NH-C(O)-, sulfo betaine, betaine, polyethylene oxide, poly(ethyleneoxide/propylene oxide/butylene oxide) grafts with any end group, H, OH, styrene sulfonate, pyridine, quaternized pyridine, alkyl-substituted pyrrolidone or pyridine, pyridine-N-oxide, imidazolinium halide, imidazolium halide, imidazol, piperidine, -OR1, -OH, -COOH alkali salt, sulfonate, ethoxy sulphate, pyrrolidone, caprolactam, phenyl-R4 or naphthalene-R5 where R4 and R5 are R1, R2, R3, sulfonic acid or its alkali salt or organic counter ion. Any mixture or these R3 groups may be used. Also, glyoxylated cationic polyacrylamides can be used. Typical polymers of choice are those containing the cationic monomer dimethylaminoethyl methacrylate (DMAEMA) or methacrylamidopropyl trimethyl ammonium chloride (MAPTAC). DMAEMA can be found in Gafquat and Gaffix VC-713 polymers from ISP. MAPTAC can be found in BASF's Luviquat PQ11 PN and ISP's Gafquat HS100.

Another group of polymers that can be used are those that contain cationic groups in the main chain or backbone. Included in this group are:
(1) polyalkylene imines such as polyethylene imine, commercially available as Lupasol from BASF. Any molecular weight and any degree of crosslinking of this polymer can be used in the present invention;
(2) ionenes having the general formula set forth as

   -[N(+)R1R2-A1-N(R5)-X-N(R6)-A2-N(+)R3R4-A3]n- 2Z-,

   as disclosed in U.S. Patent Nos. 4,395,541 and U.S. 4,597,962;
(3) adipic acid/dimethyl amino hydroxypropyl diethylene triamine copolymers, such as Cartaretin F-4 and F-23, commercially available from Sandoz;
(4) polymers of the general formula-[N(CH3)2-(CH2)x-NH-(CO)-NH-(CH2)y-N(CH3)2)-(CH2)z-O-(CH2)p]n-, with x, y, z, p=1-12, and n according to the molecular weight requirements. Examples are Polyquaternium 2 (Mirapol A-15), Polyquaternium-17 (Mirapol AD-1), and Polyquaternium-18 (Mirapol AZ-1).

Other polymers include cationic polysiloxanes and cationic polysiloxanes with carbon-based grafts with a net theoretical positive charge or equal to zero (mixture of cationic and anionic functional groups). This includes cationic end-group functionalized silicones (i.e. Polyquaternium-80). Silicones with general structure: -[-Si(R1)(R2)-O-]x-[Si(R3)(R2)-O-]y-where R1 is any alkane from C1-C25 or H with number of double bonds from 0-5,aromatic moieties, polysiloxane grafts, or mixtures thereof. R1 can also be a liquid crystalline moiety that can render the polymer thermotropic liquid crystalline properties, or the alkanes selected can result in side-chain melting. R2 can be H or CH3 and R3 can be -R1-R4, where R4 can be -NH2, -NHR1, -NR1R2, -NR1R2R6 (where R6 = R1, R2, or -CH2-COOH or its salt), -NH-C(O)-, -COOH, -COO- alkali salt, any C1-25 alcohol, -C(O)-NH2 (amide), -C(O)-N(R2)(R2')(R2"), sulfo betaine, betaine, polyethylene oxide, poly(ethyleneoxide/propylene oxide/butylene oxide) grafts with any end group, H, -OH, styrene sulfonate, pyridine, quaternized pyridine, alkyl-substituted pyrrolidone or pyridine, pyridine-N-oxide, imidazolinium halide, imidazolium halide, imidazol, piperidine, pyrrolidone, caprolactam, -COOH, -COO- alkali salt, sulfonate, ethoxy sulphate phenyl-R5 or naphthalene-R6 where R5 and R6 are R1, R2, R3, sulfonic acid or its alkali salt or organic counter ion. R3 can also be -(CH2)x-O-CH2-CH(OH)-CH2-N(CH3)2-CH2-COOH and its salts. Any mixture of these R3 groups can be selected. X and y can be varied as long as the theoretical net charge of the polymer is zero (amphoteric) or positive. In addition, polysiloxanes containing up to 5 different types of monomeric units may be used. Examples of suitable polysiloxanes are found in U.S. Patent Nos. 4,395,541 4,597,962 and 6,200,554. Another group of polymers that can be used to improve microcapsule/particle deposition are phospholipids that are modified with cationic polysiloxanes. Examples of these polymers are found in U.S. Patent No. 5,849,313, WO Patent Application 9518096A1 and European Patent EP0737183B1.

Furthermore, copolymers of silicones and polysaccharides and proteins which are commercially available as CRODASONE brand products.

Another class of polymers include polyethylene oxide-co-propyleneoxide-co-butylene oxide polymers of any ethylene oxide/propylene oxide / butylene oxide ratio with cationic groups resulting in a net theoretical positive charge or equal to zero (amphoteric). The general structure is: 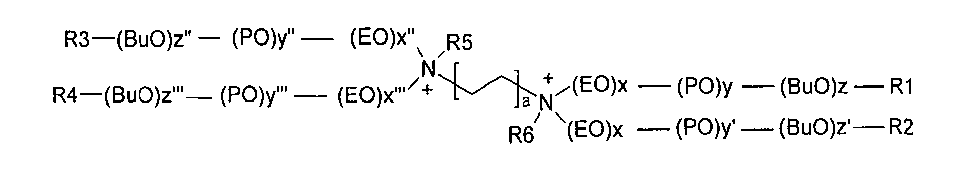 where R1,2,3,4 is -NH2, -N(R)3- X+, R with R being H or any alkyl group. R5, 6 is -CH3 or H. The value for 'a' can range from 1-100. Counter ions can be any halide ion or organic counter ion. X, Y, may be any integer, any distribution with an average and a standard deviation and all 12 can be different. Examples of such polymers are the commercially available TETRONIC brand polymers.

Suitable polyheterocyclic (the different molecules appearing in the backbone) polymers include the piperazine-alkylene main chain copolymers disclosed in Ind. Eng. Chem. Fundam., (1986), 25, pp.120-125, by Isamu Kashiki and Akira Suzuki.

Also suitable for use in the present invention are copolymers containing monomers with a cationic charge in the primary polymer chain, up to 5 different types of monomers may be used. Any co-monomer from the types listed in this specification may also be used. Examples of such polymers are poly diallyl dimethyl ammonium halides (PolyDADMAC) copolymers of DADMAC with vinyl pyrrolidone, acrylamides, imidazoles, imidazolinium halides, etc. These polymers are disclosed in Henkel EP0327927A2 and PCT Patent Application 01/62376A1. Also suitable are Polyquaternium-6 (Merquat 100), Polyquaternium-7 (Merquats S, 550, and 2200), Polyquaternium-22 (Merquats 280 and 295) and Polyquaternium-39 (Merquat Plus 3330), available from Ondeo Nalco.

Polymers containing non-nitrogen cationic monomers of the general type -CH2-C(R1)(R2-R3-R4)- can be used with:

R1 being a -H or C1-C20 hydrocarbon. R2 is a disubstituted benzene ring or an ester, ether, or amide linkage. R3 is a C1-C20 hydrocarbon, preferably C1-C10, more preferably C1-C4. R4 can be a trialkyl phosphonium, dialkyl sulfonium, or a benzopyrilium group, each with a halide counter ion. Alkyl groups for R4 are C1-C20 hydrocarbon, most preferably methyl and t-butyl. These monomers can be copolymerized with up to 5 different types of monomers. Any co-monomer from the types listed in this specification may also be used.

Substantivity of these polymers may be further improved through formulation with cationic, amphoteric and nonionic surfactants and emulsifiers, or by coacervate formation between surfactants and polymers or between different polymers. Combinations of polymeric systems, including those mentioned previously, may be used for this purpose as well as those disclosed in European Patent Application No. EP0672409.

Furthermore, polymerization of the monomers listed above into a block, graft or star (with various arms) polymers can often increase the substantivity toward various surfaces. The monomers in the various blocks, graft and arms can be selected from the various polymer classes listed in this specification and the sources below:
Encyclopedia of Polymers and Thickeners for Cosmetics, Robert Lochhead and William From, in Cosmetics & Toiletries, Vol. 108, May 1993, pp. 95-138;
Modified Starches: Properties & Uses, O. B. Wurzburg, CRC Press, 1986. Specifically, Chapters 3, 8, and 10;
U.S. Patent Nos. 6,190,678 and 6,200,554; and
PCT Patent Application WO 01/62376A1 assigned to Henkel.

Polymers, or mixtures of the following polymers:
(a) Polymers comprising reaction products between polyamines and (chloromethyl) oxirane or (bromomethyl) oxirane. Polyamines being 2(R1)N-[-R2-N(R1)-]n-R2-N(R1)2, 2HN-R1-NH2, 2HN-R2-N(R1)2 and 1H-Imidazole. Also, the polyamine can be melamine. R1 in the polyamine being H or methyl. R2 being alkylene groups of C1-C20 or phenylene groups. Examples of such polymers are known under the CAS numbers 67953-56-4 and 68797-57-9. The ratio of (chloromethyl) oxirane to polyamine in the cationic polymer ranges from 0.05-0.95.
(b) Polymers comprising reaction products of alkanedioic acids, polyamines and (chloromethyl) oxirane or (bromomethyl) oxirane. Alkane groups in alkanedioic acids C0-C20. Polyamine structures are as mentioned in (a). Additional reagents for the polymer are dimethyl amine, aziridine and polyalkylene oxide (of any molecular weight but, at least, di-hydroxy terminated; alkylene group being C1-20, preferably C2-4). The polyalkylene oxide polymers that can also be used are the Tetronics series. Examples of polymers mentioned here are known under the CAS numbers 68583-79-9 (additional reagent being dimethyl amine), 96387-48-3 (additional reagent being urea), and 167678-45-7 (additional reagents being polyethylene oxide and aziridine). These reagents can be used in any ratio.
(c) Polyamido Amine and Polyaminoamide-epichlorohydrin resins, as described by David Devore and Stephen Fisher in Tappi Journal, vol.76, No.8, pp. 121-128 (1993). Also referenced herein is "Polyamide-polyamine-epichlorohydrin resins" by W. W. Moyer and R. A. Stagg in Wet-Strength in Paper and Paperboard, Tappi Monograph Series No. 29, Tappi Press (1965), Ch.3, 33-37.

The preferred cationically charged material comprises reaction products of polyamines and (chloromethyl) oxirane. In particular, reaction products of 1H-imidazole and (chloromethyl) oxirane, known under CAS number 68797-57-9. Also preferred are polymers comprising reaction products of 1,6-hexanediamine,N-(6-aminohexyl) and (chloromethyl) oxirane, known under CAS number 67953-56-4. The preferred weight ratio of the imidazole polymer and the hexanediamine, amino hexyl polymer is from about 5:95 to about 95:5 weight percent and preferably from about 25:75 to about 75:25.

The encapsulated systems can be prepared by several processes. In one process, the polymer adjuvant material is pre-blended with the fragrance oil under slight shear to form homogenous dispersion. This mixture can then be used as core. The coating polymer is pre-formed with suitable monomers. The core material is then sheared into an aqueous solution of the coating polymer at the desired concentration. The polymerization reaction is allowed to proceed further under constant agitation at elevated temperature. The polymerization reaction is stopped after the reaction or encapsulation is complete. Encapsulation of the core material can be monitored by optical microscope.

According to the present invention, the encapsulated fragrance is well suited for a variety of applications, including wash-off products. Wash-off products are understood to be those products that are applied for a given period of time and then are removed. These products are common in areas such as laundry products, and include detergents, fabric conditioners, and the like; as well as personal care products which include shampoos, conditioner, hair colors and dyes, hair rinses, body washes, soaps and the like.

Microcapsules containing an active material, preferably perfume, suitable for use in the present compositions are described in detail in, e.g., U.S. Pat. Nos. 3,888,689; 4,520,142; 5,126,061 and 5,591,146.

The fragrances suitable for use in this invention include without limitation, any combination of fragrance, essential oil, plant extract or mixture thereof that is compatible with, and capable of being encapsulated by a polymer.

Many types of fragrances can be employed in the present invention, the only limitation being the compatibility and ability to be encapsulated by the polymer being employed, and compatibility with the encapsulation process used. Suitable fragrances include but are not limited to fruits such as almond, apple, cherry, grape, pear, pineapple, orange, strawberry, raspberry; musk, flower scents such as lavender-like, rose-like, iris-like, and carnation-like. Other pleasant scents include herbal scents such as rosemary, thyme, and sage; and woodland scents derived from pine, spruce and other forest smells. Fragrances may also be derived from various oils, such as essential oils, or from plant material such as peppermint, spearmint and the like. Other familiar and popular smells can also be employed such as baby powder, popcorn, pizza, cotton candy and the like in the present invention.

A list of suitable fragrances is provided in U.S. Patents 4,534,891, 5,112,688 and 5,145,842. Another source of suitable fragrances is found in Perfumes Cosmetics and Soaps, Second Edition, edited by W. A. Poucher, 1959. Among the fragrances provided in this treatise are acacia, cassie, chypre, cylamen, fern, gardenia, hawthorn, heliotrope, honeysuckle, hyacinth, jasmine, lilac, lily, magnolia, mimosa, narcissus, freshly-cut hay, orange blossom, orchids, reseda, sweet pea, trefle, tuberose, vanilla, violet, wallflower, and the like. Those with skill in the art appreciate that fragrance formulations are frequently complex mixtures of many fragrance ingredients. A perfumer commonly has several thousand fragrance chemicals to work from. Those with skill in the art appreciate that the present invention may contain a single ingredient, but it is much more likely that the present invention will comprise at least eight or more fragrance chemicals, more likely to contain twelve or more and often twenty or more fragrance chemicals. The present invention also contemplates the use of complex fragrance formulations containing fifty or more fragrance chemicals, seventy five or more or even a hundred or more fragrance chemicals in a fragrance formulation.

The level of fragrance in the microcapsule product varies from about 0.1 to about 95 weight percent (%), preferably from about 1 to about 80 weight % and most preferably from about 5 to about 60 weight %. In addition to the fragrance, other material can be used in conjunction with the fragrance and are understood to be included.

As used herein olfactory effective amount is understood to mean the amount of compound in perfume compositions the individual component will contribute to its particular olfactory characteristics, but the olfactory effect of the fragrance composition will be the sum of the effects of each of the fragrance ingredients. Thus the compounds of the invention can be used to alter the aroma characteristics of the perfume composition by modifying the olfactory reaction contributed by another ingredient in the composition. The amount will vary depending on many factors including other ingredients, their relative amounts and the effect that is desired.

As noted above, the fragrance may also be combined with a variety of solvents which serve to increase the compatibility of the various material, increase the overall hydrophobicity of the blend, influence the vapor pressure of the material, or serve to structure the blend. Solvents performing these functions are well known in the art and include mineral oils, triglyceride oils, silicone oils, fats, waxes, fatty alcohols, diisodecyl adipate and diethyl phthalate among others.

A common feature of many encapsulation processes is that they require the fragrance material to be encapsulated to be dispersed in aqueous solutions of polymers, pre-condensates, surfactants, and the like prior to formation of the capsule walls. Therefore, material having low solubility in water, such as highly hydrophobic material are preferred, as they will tend to remain in the dispersed perfume phase and partition only slightly into the aqueous solution. Fragrance material with Clog P values greater than 1, preferably greater than 3, and most preferably greater than 5 will thus result in micro-capsules that contain cores most similar to the original composition, and will have less possibility of reacting with material that form the capsule shell.

One object of the present invention is to deposit capsules containing fragrance cores on desired substrates such as cloth, hair, and skin during washing and rinsing processes. Further, it is desired that, once deposited, the capsules release the encapsulated fragrance either by diffusion through the capsule wall, via small cracks or imperfections in the capsule wall caused by drying, physical, or mechanical means, or by large-scale rupture of the capsule wall. In each of these cases, the volatility of the encapsulated perfume material is critical to both the speed and duration of release, which in turn control consumer perception. Thus, fragrance chemicals which have higher volatility as evidenced by normal boiling points of less than 250°C, preferably less than about 225°C are preferred in cases where quick release and impact of fragrance is desired. Conversely, fragrance chemicals that have lower volatility (boiling points greater than 225°C) are preferred when a longer duration of aroma is desired. Of course, fragrance chemicals having varying volatility may be combined in any proportions to achieve the desired speed and duration of perception.

The present active material compositions may further comprise one or more malodour counteractant at a level preferably less than about 70 weight %, more preferably less than about 50 weight % of the composition. The malodour counteractant composition serves to reduce or remove malodor from the surfaces or objects being treated with the present compositions. The malodour counteractant composition is preferably selected from uncomplexed cyclodextrin, odor blockers, reactive aldehydes, flavanoids, zeolites, activated carbon, and mixtures thereof. Compositions herein that comprise odor control agents can be used in methods to reduce or remove malodor from surfaces treated with the compositions.

Specific examples of malodour counteractant components useful in aminoplast microencapsulate used in the composition and process of our invention are as follows:
Malodour Counteractant Component Group I:
1-cyclohexylethan-1-yl butyrate;
1-cyclohexylethan-1-yl acetate;
1-cyclohexylethan-1-ol;
1-(4'-methylethyl)cyclohexylethan-1-yl propionate; and
2'-hydroxy-1'-ethyl(2-phenoxy)acetate
each of which compound is marketed under the trademark VEILEX by International Flavors & Fragrances Inc., New York, N.Y., U.S.A. Malodour Counteractant Component Group II, as disclosed in U.S. Patent 6,379,658:
β-naphthyl methyl ether;
β-naphthyl ketone;
benzyl acetone;
mixture of hexahydro-4,7-methanoinden-5-yl propionate and hexahydro-4,7-methanoinden-6-yl propionate;
4-(2,6,6-trimethyl-2-cyclohexen-1-yl)-3-methyl-3-buten-2-one;
3,7-dimethyl-2,6-nonadien-1-nitrile;
dodecahydro-3a,6,6,9a-tetramethylnaphtho(2,1-b)furan;
ethylene glycol cyclic ester of n-dodecanedioic acid;
1-cyclohexadecen-6-one;
1-cycloheptadecen-10-one; and
corn mint oil.

In addition to the fragrance material in the present invention contemplates the incorporation of solvent material into the microcapsule product. The solvent material is a hydrophobic material that are miscible in the fragrance material used in the present invention. The solvent material serves to increase the compatibility of various active material, increase the overall hydrophobicity of the blend, influence the vapor pressure of active material, or serve to structure the blend. Suitable solvents are those having reasonable affinity for the fragrance chemicals and a ClogP greater than 2.5, preferably greater than 3.5 and most preferably greater that 5.5. Suitable solvent material include, but are not limited to triglyceride oil, mono and diglycerides, mineral oil, silicone oil, diethyl phthalate, polyalpha olefins, castor oil and isopropyl myristate. In a preferred embodiment the solvent material are combined with fragrance material that have ClogP values as set forth above. It should be noted that selecting a solvent and fragrance with high affinity for each other will result in the most pronounced improvement in stability. Appropriate solvents may be selected from the following non-limiting list:
- Mono-, di- and tri-esters, and mixtures thereof, of fatty acids and glycerine. The fatty acid chain can range from C4-C26. Also, the fatty acid chain can have any level of unsaturation. For instance capric/caprylic triglyceride known as Neobee M5 (Stepan Corporation). Other suitable examples are the Capmul series by Abitec Corporation. For instance, Capmul MCM.
- Isopropyl myristate
- Fatty acid esters of polyglycerol oligomers: R2CO-[OCH2-CH(OCOR1)-CH2O-]n, where R1 and R2 can be H or C4-26 aliphatic chains, or mixtures thereof, and n ranges between 2 - 50, preferably 2-30.
- Nonionic fatty alcohol alkoxylates like the Neodol and Dobanol surfactants by Shell Corporation or the BioSoft surfactants by Stepan or Utensil by BASF. The alkoxy group being ethoxy, propoxy, butoxy or mixtures thereof. In addition, these surfactants can be end-capped with methyl groups in order to increase their hydrophobicity.
- Di- and tri-fatty acid chain containing nonionic, anionic and cationic surfactants, and mixtures thereof.
- Fatty acid esters of polyethylene glycol, polypropylene glycol, and polybutylene glycol, or mixtures thereof.
- Polyalphaolefins such as the ExxonMobil PureSym^{™} PAO line
- Esters such as the ExxonMobil PureSyn^{™} Esters
- Mineral oil
- Silicone oils such polydimethyl siloxane and polydimethylcyclosiloxane
- Diethyl phthalate
- Di-isodecyl adipate.

While no solvent is needed in the core, in one embodiment the level of solvent in the core of the microcapsule product weight percent should be greater than about 20 weight %, preferably greater than about 50 weight % and most preferably less than about 50 weight %. In addition to the solvent it is preferred that higher ClogP fragrance materials are employed. It is preferred that greater than about 25 weight %, preferably greater than 50 weight % and more preferably greater than about 80 weight % of the fragrance chemicals have ClogP values of greater than about 2.0, preferably greater than about 3.0 and most preferably greater than about 3.5. Those with skill in the art will appreciate that many formulations can be created employing various solvents and fragrance chemicals. The use of high ClogP fragrance chemicals will require a lower level of hydrophobic solvent than fragrance chemicals with lower ClogP to achieve similar stability. As those with skill in the art will appreciate, in a highly preferred embodiment high ClogP fragrance chemicals and hydrophobic solvents comprise greater than about 80 weight %, preferably more than about 90 weight % and most preferably greater than 99 weight % of the fragrance composition.

A common feature of many encapsulation processes is that they require the fragrance material to be encapsulated to be dispersed in aqueous solutions of polymers, pre-condensates, surfactants, and the like prior to formation of the microcapsule walls.

In order to provide the highest fragrance impact from the fragrance encapsulated microcapsules deposited on the various substrates referenced above, it is preferred that material with a high odor-activity be used. Material with high odor-activity can be detected by sensory receptors at low concentrations in air, thus providing high fragrance perception from low levels of deposited microcapsules. This property must be balanced with the volatility as described above. Some of the principles mentioned above are disclosed in U.S. Patent No. 5,112,688.

As described herein, the present invention is well suited for use in a variety of well-known consumer products such as liquid and powder detergent, laundry detergent and fabric softeners, liquid dish detergents, automatic dish detergents, as well as hair shampoos and conditioners, deodorants and antiperspirants. These products employ surfactant and emulsifying systems that are well known. For example, fabric softener systems are described in U.S. Patents 6,335,315, 5,674,832, 5,759,990, 5,877,145, 5,574,179; 5,562,849, 5,545,350, 5,545,340, 5,411,671, 5,403,499, 5,288,417, and 4,767,547, 4,424,134. Liquid dish detergents are described in U.S. Patents 6,069,122 and 5,990,065; automatic dish detergent products are described in U.S. Patents 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562. Liquid laundry detergents which can use the present invention include those systems described in U.S. Patents 5,929,022, 5,916,862, 5,731,278, 5,565,145, 5,470,507, 5,466,802, 5,460,752, 5,458,810, 5,458,809, 5,288,431,5,194,639, 4,968,451, 4,597,898, 4,561,998, 4,550,862, 4,537,707, 4,537,706, 4,515,705, 4,446,042, and 4,318,818. Shampoo and conditioners that can employ the present invention include those described in U.S. Patents 6,162,423, 5,968,286, 5,935,561, 5,932,203, 5,837,661, 5,776,443, 5,756,436, 5,661,118, 5,618,523, 5,275,755, 5,085,857, 4,673,568, 4,387,090 and 4,705,681. All of the above mentioned U.S. Patents.

All U.S. Patents and patent applications cited herein are incorporated by reference as if set forth herein in their entirety.

The following examples are provided as specific embodiments of the present invention. These and additional modifications and improvements of the present invention may also be apparent to those with ordinary skill in the art. The particular combinations of elements described and illustrated herein are intended to represent only a certain embodiment of the present invention and are not intended to serve as limitations of alternative articles within the spirit and scope of the invention.

### Example 1

### Preparation of Fragrance Core with a Polymeric Adjuvant

The fragrance used in this example was Fresh Zion, a commercially available fragrance from IFF. The polymeric adjuvant was a polybutadiene homopolymer commercially available under the tradename Poly bd^{®} R45HTLO, from the Sartomer Company, Inc., Exton, PA. This polymer is a liquid at room temperature and is readily blended with Fresh Zion fragrance at different ratios.

60 g of the Poly bd^{®} R45HTLO was blended with 105 g of the Fresh Zion fragrance and 45 g of NEOBEE M5 oil, commercially available from Stephan Company, Northfield, IL, in a 16 Oz jar. In a separate reaction vessel, 34 g of a copolymer of acryl amide and acrylic acid was first dispersed in 300 ml of water together with 18 g of a methylated melamine-formaldehyde resin. These two components were allowed to react under acidic conditions for the desired amount of time at room temperature. The fragrance core material was then added to polymer solution. The system was subject to homogenization with an overhead mixture at about 800 rpm to promote the formation of capsules by the pre-formed polymer. Curing of the polymeric layer around the fragrance encapsulate was achieved by increasing the temperature to above 80°C and maintaining the temperature for desired amount of time. The slurry was collected for future use.

Capsule size analysis was performed using a Malvern Masterziser 2000E indicated the particle revealed that the volume-averaged capsule size is 65 micron. The TGA thermogram of the same sample is given in Figure I and was contrasted with that of a smaller capsule.

### Comparative Example 1

This example illustrates the critical role of the liquid polymer adjuvant in making larger capsules. This is demonstrated by repeating the capsule making process in Example 1. However, the liquid polymer adjuvant was replaced with NEOBEE M5 oil to create an oil core that contains 50% Neobee oil and 50% Fresh Zion fragrance (IFF). Some larger capsules with an average particle size > 20 micron were formed initially under lower shear conditions, but these capsules collapsed rapidly during the curing process.

This example clearly demonstrates the unique function of the liquid polymer adjuvant in enabling the formation and further stabilization of larger capsules.

### Example 2

### Use of another polymer adjuvant in preparing stable larger capsules

The fragrance used was the Healing fragrance, commercially available from IFF. The core was prepared by blending 21 g of Krasol^{®} LBH 10000, commercially available Sartomer Company, Inc, 84, Neobee M5 oil and 105 g of the fragrance oil. The procedure outlined in Example I was repeated and stable larger capsule was obtained. The measured capsule size was 58 micron using a Malvern Masterziser 2000E.

### Comparative Example 2

In this example, the Krasol^{®} LBH 10000 (21 g) was replaced with Neobee M5 oil and the capsule making process described in Example 2 was repeated. No stable large capsules were obtained.

It is quite evident the presence of liquid polymer adjuvant is necessary in creating and stabilizing larger capsules.

### Example 3

### Application of present innovation in producing lager fragrance capsules using a polymer adjuvant and a densification agent

To prepare the larger fragrance capsule, 105 g of Orchard Freshness fragrance, commercially available from IFF, was blended with 42 g of Neobee M5 oil, 54 g of the polymeric adjuvant, Poly bd^{®} R20 LM and 9 of Uvinul^{®} TiO₂. The mixture was sheared mixed and encapsulated the same way as in Example 1.

Capsule size analysis was performed using a Malvern Masterziser 2000E indicated the particle revealed that the volume-averaged capsule size is 95 micron.

### Application of the present innovation in producing more larger fragrance capsules Examples 4-10

Several larger capsules were prepared using the current invention using the process outlined in Example 1. The core loading was 35% and fragrance, Fresh Zion, loading was 17.5% in all cases.

### Example 4

Capsule slurry in Example 4 contained a core consisted of 50% Neobee M5, 10% Polymeric adjuvant, Stantive^{™} OMA-1, from CasChem, Bayonne, NJ. The capsules were cured at above 80°C.

### Example 5

Capsule slurry in Examples 5 contained a core composed of 50% FZ fragrance and 50% of polymeric adjuvant, Poly bd^{®} R45 HTLO. The capsules were cured at above 80°C.

### Example 6

The slurry in Examples 6 contained a core consists of 50% FZ fragrance, 40% Poly bd^{®} R45HTLO, and 10% Neobee M5. The capsules were cured at above 80°C.

### Example 7

The sample in Examples 7 contained a core containing 50% FZ fragrance and 10% Poly bd^{®} R45 HTLO, and 40% Neobee M5. The capsules were cured at above 80°C.

### Examples 8

Capsule slurry in Examples 8 contained a core composed of 50% FZ fragrance, 30% of polymeric adjuvant, Poly bd^{®} R45HTLO, and 20% Neobee M5. The capsules were cured at above 90°C.

### Example 9

The slurry in Examples 9 contained a core consists of 50% FZ fragrance, 25% Poly bd^{®} R20LM, and 25% Neobee M5. The capsules were cured at above 90°C.

### Example 10

The sample in Examples 10 was prepared with a core containing 50% FZ fragrance and 15% Poly bd^{®} R20LM, and 35% Neobee M5. The capsules were cured at above 90°C.

### Example 11

### Enhanced performance can be achieved by the larger capsules produced by the current invention

To conduct the stability and performance evaluations, the smaller melamine-formaldehyde capsule slurry (made by Celessence International Ltd., West Molesey, Surrey, UK) that contains approximately 35% by weight of the fragrance and 57% by weight of water was used as bare (uncharged) capsules in the following examples. To make the melamine-formaldehyde capsule slurry, a copolymer of poly acrylamide and acrylic acid was first dispersed in water together with a methylated melamine-formaldehyde resin. Fragrance was then added into the solution with high speed shearing to form small droplets. Curing of the polymeric film over the fragrance droplets as capsule wall affected by increasing the solution pH to polymerize the polymers followed by heating the solution from 50 to 85°C. The volume-averaged capsule size was about 8 microns. The volume-averages sizes of the larger capsules prepared according to the current invention were larger than 50 micron. Both capsule systems contained 17.5% Fresh Zion fragrance (IFF) and were prepared at the same process temperature. The analytical solutions were prepared by blending the capsule slurry into a model rinse conditioner base that contained about 10% surfactant. The concentration of the fragrance in the base was 1% neat equivalent in all cases. The samples were placed in a 35°C oven for extend period of time. Aliquot of samples were taken out periodically and amount of fragrance leached out was analyzed according to a method by a U.S. Patent Application 11/034,593.

The results obtained on the leaching of fragrance from two fragrance slurries consisting of large capsules and a capsule slurry containing smaller capsules are given in Table 1 below. The larger capsule slurries prepared in Examples 1 and 4 are used in this example.

As it can clearly be seen the leaching of aromatic chemical is retarded significantly in the larger capsules. The leaching performance of the capsules was improved by almost 100 percent.

**Table 1: Comparison of the fragrance release from larger and smaller capsules in a 9% cationic surfactant solution**

| Capsule type | Fragrance release (%) after 4 weeks | Fragrance release (%) after 8 weeks | Improvement factor (% ) ^{a} after 8 weeks |
|---|---|---|---|
| Smaller capsule | 12 | 26 | |
| Large capsule Example 1 | 5 | 13 | 100 |
| Large capsule Example 4 | 6 | 17 | 52 |

| | | | |
|---|---|---|---|
| Note: ^{a}The improvement factor was calculated by: (release % of smaller capsule - release % of larger capsule)/ release % of larger capsule. | | | |

### Example 12

### Demonstration of the effectiveness of large capsules containing occluded fragrance materials in a more concentrated surfactant solution

It is commonly known that concentrated surfactant solution such as detergent interacts strongly with fragrance capsules and is very inductive towards the leaching of encapsulated aroma chemicals. Thus for better stability and performance, it is highly desirable to have a capsule system in which the leaching of aroma chemical was minimized. This has been achieved using the large capsules prepared with our invention.

Table Two tabulates the results obtained using the two larger Fresh Zion capsules and a smaller capsule with when the capsule slurry was blended into a 24% cationic surfactant solution. The results clearly shown that the leaching of occluded fragrance chemicals had been significantly reduced using the large capsules.

**Table 2: Comparison of the fragrance release from larger and smaller capsules in a 24% cationic surfactant solution**

| Capsule type | Fragrance release (%) after 4 weeks | Fragrance release (%) after 8 weeks | Fragrance release (%) after 12 weeks | Improvement factor (% )^{a} after 12 weeks |
|---|---|---|---|---|
| Smaller capsule | 12 | 25 | 42 | |
| Large Capsule Example 1 | 11 | 16 | 20 | 110 |
| Large Capsule Example 4 | 9 | 12 | 14 | 200 |

| | | | | |
|---|---|---|---|---|
| Note: ^{a} Calculated by the same procedure as outlined in Table 1. | | | | |

### Example 13

### Demonstration of the superior perfumery performance obtained using the larger capsules as compared to the use of smaller capsules

All capsule slurries were prepared using formulations that contained 35% core loading. The core contained 50% Fresh Zion fragrance with the polymeric adjuvant and Neobee M5 accounting for the other 50%. Lager fragrance capsules prepared in examples 4, 5, 6, and 7 are used in this example.

To establish the superior performance of the large capsules, the capsule slurry was blended into a model rinse conditioner solution that contains 9% cationic surfactant. The fragrance load was 0.3% neat equivalent. For comparison, a similar solution was prepared using a smaller capsule with a volume average capsule size of about 8 to 10 microns. The samples were subject to an accelerated storage test at 35 °C for 2 weeks and the perfumery benefit of the capsules was evaluated by conducting a laundry experiment using accepted experimental protocols. Terry towels were used for the washing experiments and were air-dried overnight before being evaluated by panel of 12 judges. The fragrance intensity is rated from a scale ranging from 0 to 30. A numerical value of 5 would suggest the fabric only produce very week intensity while a value of 30 indicates the subject generate a strong smell. The results are in Table 3.

**Table 3: Contrasting the Sensory performance of large capsules with that of smaller capsules**

| Samples | Pre-rubbing intensity | Post-rubbing intensity | I ₚₒₛₜ/ₚᵣₑ | Ratio of larger over smaller in post-rubbing |
|---|---|---|---|---|
| Blank | 5.2 | 4.2 | | |
| Smaller capsule | 6.1 | 11.1 | 1.82 | 1 |
| Large capsule Example 4 | 7.1 | 16.1 | 2.26 | 1.45 |
| Large capsule Example 5 | 6.9 | 16.5 | 2.39 | 1.49 |
| Large capsule 6 | 6.8 | 17.1 | 2.51 | 1.54 |
| Large capsule Example 7 | 7.2 | 17.0 | 2.36 | 1.53 |

The larger fragrance capsules produced consistently higher fragrance intensity at the pre-rubbing and post-rubbing stages stage. The increase in fragrance intensity is much more pronounced in the post rubbing stage. This demonstrates that the larger fragrance capsules prepared with the current invention are able to retain the fragrance much more effectively and are capable of delivering the full consumer benefits of the fragrance products.

Because the measured sensory performance was determined by several complex variables including fragrance leaching, capsule deposition, capsule breaking, the excellent sensory performance displayed by the larger capsule described in this invention is quite remarkable. It provides means for engineering fragrance capsules in demanding applications including concentrated surfactant solutions.

### Example 12

### Demonstration of the robust storage stability, favorable fragrance release profile and superior perfumery performance of the large capsules

All capsule slurries were prepared using formulations that contained 35% core loading and details are given in examples 8 , 9, and 10.

To conduct the study, the capsule slurry was blended into a model rinse conditioner solution that contains 24 % cationic surfactant. The fragrance loading was at 0.3% neat equivalent in all cases. The samples were aged at 37 °C for up to 16 weeks in a temperature controlled oven. Laundry and sensory experiments were conducted using fresh samples and samples that have been aged for 16 weeks according the experimental protocols as outlined in Example 11 except that the towels were machined dried in these experiments. The results are given in Table Four and Five.

**Table 4: Contrasting the Sensory performance of large capsules with that of smaller capsules using fresh samples**

| Samples | Pre-rubbing intensity | Post-rubbing intensity | Iₚᵣₑ/Iₚₒₛₜ | Ratio of larger over smaller in post-rubbing |
|---|---|---|---|---|
| Blank | 2.9 | 3.6 | | |
| Smaller capsule | 6.4 | 14.0 | 0.46 | 1.0 |
| Large capsule Example 8 | 16.4 | 16.9 | 0.98 | 1.21 |
| Large capsule Example 9 | 17.0 | 18.7 | 0.91 | 1.31 |
| Large capsule Example 10 | 17.1 | 19.5 | 0.88 | 1.39 |

The results in Table four indicated that the larger capsules have significant higher perfumery intensity than the smaller capsule in both the pre-rubbing and post-rubbing stage. The contrasts in the pre-rubbing intensities between the smaller and larger capsules are more drastic. The higher pre-rubbing intensities of the larger capsules are quite remarkable and are indicative of a different and favorable fragrance release mechanism than that in the smaller capsule. These important performance attributes clearly demonstrated the large capsules prepared by the current invention have significant application and consumer benefits compared with smaller capsules.

Sensory results of the larger capsules after extended storage are given in Table Five. The results indicated the larger capsules have excellent storage stability and have maintained their excellent consumer benefits after four months of storage. The performance differentials between the large and smaller capsules are even more transparent in the post-rubbing stage.

**Table 5: Contrasting the Sensory performance of large capsules with that of smaller capsules using fresh samples after four months of storage at 37 °C**

| Samples | Pre-rubbing intensity | Post-rubbing intensity | Iₚᵣₑ/Iₚₒₛₜ | Ratio of larger over smaller in post-rubbing |
|---|---|---|---|---|
| Blank | 2.7 | 4.0 | | |
| Smaller capsule | 7.3 | 10.5 | 0.69 | 1.0 |
| Large capsule Example 8 | 10.9 | 15.2 | 0.72 | 1.45 |
| Large capsule Example 9 | 16.6 | 21.9 | 0.76 | 2.09 |
| Large capsule Example 10 | 16.2 | 22.1 | 0.73 | 2.11 |

The results in Examples 11 and 12 suggest that the larger capsules outperformed smaller capsules in both dilute and concentrated surfactant media. The larger capsules are particularly suited for application in harsh application environments where concentrated surfactant solutions are used.

### Example 13

### Demonstration of the performance of the large capsules in a predominately anionic liquid detergent solution

Two capsule slurries were prepared with Orchard Fresheness Plus (OFP) fragrance, commercially available from IFF. The slurries contained 35% core load and the core contained 50% OFP fragrance with the polymeric adjuvant and Neobee M5 accounting the other 50%. The amount of polymeric adjuvant, Krasol^{®} LBH 10000 (from the Sartomer Company, Exton, PA) at is 20% in Sample 13A. Sample 13B contained 20% polymeric adjuvant, Poly bd^{®} R20LM. Both samples were cured at above 90 C. A smaller OFP capsule was also prepared with the procedure outlined in Example 11.

For sensory evaluations, a commercial detergent solution, Tide, (P&G, Cincinnati, OH) was used in all experiments. The tide solution contained about 25% surfactants that contained mainly anionic sulfonate. A market product, Tide with Febreze, (P&G, Cincinnati, OH), which is fragranced product, was also used for comparison. The fragrance was mixed into the liquid detergent solution to give a fragrance loading of 0.125%. Laundry and sensory experiments were conducted according the experimental protocols as outlined in Example V except that the towels were machined dried in these experiments. The results are presented in Table six.

**Table 6: Contrasting the Sensory performance of large capsules with that of smaller capsules in liquid detergent**

| Samples | Pre-rubbing intensity | Post-rubbing intensity | I_{pre/}Iₚₒₛₜ | Ratio to market product in pre-rubbing | Ratio to market product in post-rubbing |
|---|---|---|---|---|---|
| Blank | 6.5 | 6.1 | | | |
| Market product | 8.1 | 7.2 | | | |
| Smaller capsule | 7.5 | 15.4 | 0.49 | 0.93 | 2.14 |
| Large capsule Example 13A | 14.3 | 14.2 | 1.01 | 1.78 | 1.97 |
| Large capsule Example 13B | 15.5 | 15.9 | 0.98 | 1.91 | 2.20 |

It was quite apparent that the large capsules offer a 100% improvement in perfumery intensity over the market product at both the pre-rubbing and post-rubbing stages.

The samples containing larger capsules also generated twice the amount of pre-rubbing intensity when compared with a smaller capsule.

The favorable perfumery release profile exhibited by the large capsules provides significant improvement over both the smaller capsules and market products.

The larger capsule has the potential of true continuous and attrition free fragrance release system.

Storage tests were conducted at 37°C using the large capsules. The ensuring sensory tests indicated the larger capsule retained its excellent performance and the results are given in Table 7. As it can be clearly seen the large capsules are able to retain their good perfumery performance after 2 weeks at 37°C. They also provide superior consumer benefits over the market product at both room temperature and 37 °C.

**Table 7: Sensory performance of large capsules before and after storage**

| Samples | Pre-rubbing intensity | Post-rubbing intensity | Ratio to market product in pre-rubbing | Ratio to market product in post-rubbing |
|---|---|---|---|---|
| Blank | 2.5 | 3.7 | | |
| Market product 2 weeks at RT | 3.6 | 5.3 | | |
| Market product 2 weeks at 37 °C | 3.5 | 2.5 | | |
| Large capsule Example 13A after 2 weeks at RT | 10.9 | 15.2 | 3.02 (RT) | 2.87 (RT) |
| Large capsule Example 13A after two weeks at 37 °C | 12.1 | 13.6 | 3.46 (37) | 5.44 (37) |

### Example 14

### Performance of the large capsules in a hair conditioner

The fragrance, Brillance or Fresh Zion, from IFF was used to prepare capsule slurry with a 35% core. The core contained 50% neat fragrance, 20% polymeric adjuvant, Poly bd^{®} R45HTLO, and 30% Neobee M5. A model conditioner base consists of 5 to 8 % surfactant was prepared for the evaluation. The surfactant was mainly cationic in nature. The capsule was blended into the surfactant to give a fragrance loading of 0.125% neat equivalent.

The performance of the capsules was evaluated using Mannequin heads using accepted experimental protocols. The sensory benefits of the samples were evaluated by a panel of 10 judges. The perfumery intensity was assigned a value from 0 to 10 with 10 being extremely strong and 0 being no sensation. The fragrance intensity was evaluated before and after the Mannequin head was combed and the results are tabulated in Table eight.

**Table 8: Sensory performance of large capsules in hair conditioner base**

| Samples | Pre-coming intensity | Post-combing intensity | Iₚₒₛₜ/Iₚᵣₑ₋ coming |
|---|---|---|---|
| Neat | 0.25 | 0.25 | |
| Large Brillance capsule | 0.88 | 5.25 | 6.0 |

The benefit of larger capsules was clearly demonstrated by their superior intensity over that of the neat sample. The dramatic increase in perfumery intensity when the hair was combed indicates that the large capsule can act retain the aromatic chemicals and release its content when minor force is applied.

The performance of the large capsules was further compared with a commercial hair conditioner, Pantene (P&G, Cincinnati, OH). The results are given in Table 9.

**Table 9: Sensory performance of large capsules in hair conditioner base compared with a marker product**

| Samples | Pre-coming intensity | Post-combing intensity | Ratio to market product in post-combing |
|---|---|---|---|
| Pantene Market product | 0.36 | 0.36 | |
| Large Fresh Zion capsule | 1.07 | 3.93 | 10.9 |

The larger capsule generated much stronger fragrance intensity than the market product at both pre-combing and post-combing stages.

The release of encapsulated fragrance from the larger capsule is quite substantial as the fragrance intensity increased almost four hundred percent when the hair is combed.

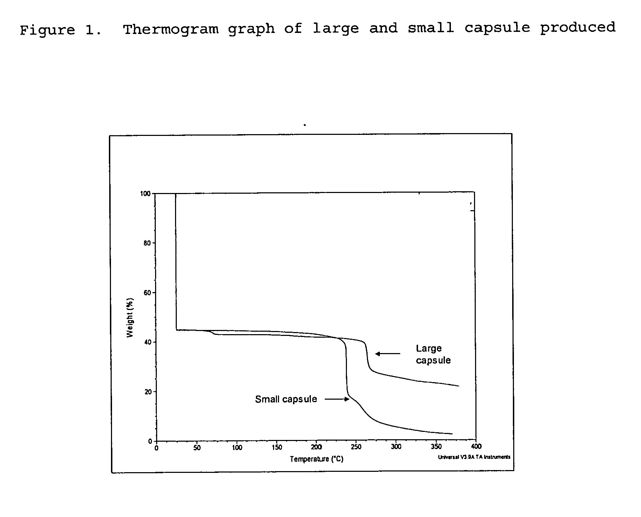

## Claims

1. A capsule particle comprising a combination of an active ingredient, a polymer adjuvant and an encapsulating polymeric material.

2. The capsule particle of claim 1 wherein the polymer adjuvant is selected from a polybutadiene homopolymer, a polybutadiene homopolymer derivatives and mixtures thereof, and optionally or preferably wherein the polybutadiene polymer can have molecular weight range from about 500 to about 15000.

3. The capsule particle of claim 2 wherein the polybutadiene polymer is a linear polymer or a branched polymer.

4. The capsule particle of claim 2 or claim 3 wherein the polybutadiene polymer contains functional groups selected from the group consisting of hydroxyl, carboxylate, isocyanante, anhydride, epoxide and mixtures thereof.

5. The capsule particle of claim 4 wherein the functional groups are primary or are secondary.

6. The capsule particle of claim 1, or of any of claims 2 to 5, wherein the polymer adjuvant is selected from the group consisting of ethylene/propylene/styrene copolymer, butylene/ethylene/styrene copolymer and mixture thereof, and optionally or preferably wherein the polymer adjuvant is pre-dissolved in a solvent selected from the group consisting of mineral, hydrogenated polyisobutene, isopropyl palmitate, C12-15 alkyl benzoate, isohexadecane, isododecane, isononly isononanoate and mixtures thereof.

7. The capsule particle of claim 1, or of any of claims 2 to 6, wherein the active ingredient is a fragrance oil.

8. The capsule particle of claim 1, or of any of claims 2 to 7, additionally comprising a hydrophobic material selected from the group consisting of TiO2, FeO2, SiO2 and mixture thereof.

9. The capsule particle of claim 1, or of any of claims 2 to 8, wherein the polymer adjuvant is present up to about 90% in the capsule particle; or
(ii) 50%; or
(iii) 30%.

10. The capsule particle of claim 1, or of any of claims 2 to 9, wherein the encapsulating polymeric material is selected from the group consisting of a vinyl polymer, an acrylate polymer, an acrylate acrylamide copolymer, melamine-formaldehyde polymer, urea-formaldehyde polymer and mixtures thereof to form a polymer encapsulated fragrance.

11. The capsule particle of claim 1, or of any of claims 2 to 10, wherein the encapsulating polymeric material is a crosslinked network of polymers comprising a melamine-formaldehyde: acrylamide-acrylic acid copolymer wherein the mole ratio is in the range of from about 9:1 to about 1:9; and optionally or preferably wherein the molar ratio of melamine-formaldehyde: acrylamide-acrylic acid copolymer is in the range of from about 5:1 to about 1:5;or from about 2:1 to about 1:2.

12. The capsule particle of claim 1, or of any of claims 2 to 11, wherein the capsule particle is further coated with a polymer coating material, and optionally or preferably wherein the polymer coating material is cationically charged.

13. The capsule particle of claim 12 wherein the polymer coating material is selected from the group consisting of polysaccharides, cationically modified starch, cationically modified guar, polysiloxanes, poly diallyl dimethyl ammonium halides, copolymers of poly diallyl dimethyl ammonium chloride and vinyl pyrrolidone, acrylamides, imidazoles, imidazolinium halides, imidazolium halides, poly vinyl amine, copolymers of poly vinyl amine and N-vinyl formamide, and mixtures thereof.

14. The capsule particle of claim 1, or of any of claims 2 to 13, wherein the active ingredient is selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants, and mixtures thereof.

15. A process for preparing a capsule particle comprising the steps of:
mixing a active ingredient and a polymer adjuvant to provide an oil phase;
providing an aqueous phase polymer solution comprising a crosslinked network of polymers;
emulsifying the oil phase into the aqueous phase polymer solution to form an emulsion;
curing the emulsion; and
providing a capsule particle with a diameter from about 5 to about 2000 microns.

16. The process of claim 15 wherein the capsule particle diameter is from about 10 to about 500 micron, or from about 20 to about 100 micron.

17. The process of claim 15 or claim 16 wherein the polymer adjuvant is present up to about 90% in the capsule particle, or wherein the polymer adjuvant is present up to about 50%, or wherein the polymer adjuvant is present up to about 30%.

18. The process of claim 15, or claim 16, or claim 17, wherein the oil phase is present from about:
(i) 1 to about 60% and the aqueous polymer solution is present from about 1 to about 20 %; or
(ii) 10% to about 45% and the aqueous polymer solution is present from about 2 to about 10 %.

19. The process of claim 15, or any of claims 16 to 18, wherein the polymer adjuvant is a polybutadiene homopolymer, and optionally or preferably wherein the polybutadiene polymer can have molecular weight range from about 500 to about 15000 and have narrow or broad molecular weight distribution.

20. The process of claim 15, or of any of claims 16 to 19, wherein the polymer adjuvant is selected from the group consisting of ethylene/propylene/styrene copolymer, butylene/ethylene/styrene copolymer and mixture thereof.

21. The process of claim 15, or of any of claims 16 to 20, wherein the polymer adjuvant is pre-dissolved in a solvent selected from the group consisting of mineral, hydrogenated polyisobutene, isopropyl palmitate, C12-15 alkyl benzoate, isohexadecane, isododecane,isononly isononanoate and mixtures thereof.

22. The process of claim 15, or of any of claims 16 to 21, wherein the emulsion is cured at a temperature from about 60 to about 180°C.

23. The process of claim 15, or of any of claims 16 to 22, wherein
(i) the crosslinked network of polymers is selected from the group consisting of a vinyl polymer, an acrylate polymer, an acrylate acrylamide copolymer, melamine-formaldehyde polymer, urea-formaldehyde polymer and mixtures thereof to form a polymer encapsulated fragrance; or
(ii) the crosslinked network of polymers is a crosslinked network of polymers comprising a melamine-formaldehyde: acrylamide-acrylic acid copolymer wherein the mole ratio is in the range of from about 9:1 to about 1:9, or is in the range of from about 5:1 to about 1:5, or is in the range of from about 2:1 to about 1:2.

24. The process of claim 15, or of any of claims 16 to 23 wherein comprising the additional step of coating capsule particle with a polymer coating material, and preferably or optionally the polymer coating is cationically charged, and preferably or optionally the polymer coating material being selected from the group consisting of polysaccharides, cationically modified starch, cationically modified guar, polysiloxanes, poly diallyl dimethyl ammonium halides, copolymers of poly diallyl dimethyl ammonium chloride and vinyl pyrrolidone, acrylamides, imidazoles, imidazolinium halides, imidazolium halides, poly vinyl amine, copolymers of poly vinyl amine and N-vinyl formamide to the surface of the polymer encapsulated fragrance to form a cationically coated polymer encapsulated material.

25. The process of claim 15, or any of claims 16 to 24, wherein the active ingredient selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants, and mixtures thereof.

26. A consumer product comprising the capsule particle prepared according to the process of any of claims 15 to 25, and optionally or preferably wherein the consumer product is selected from the group consisting rinse conditioner, detergents, industrial and institutional cleaners, personal care, shampoo, conditioner, and body wash.
